# EUROPEAN PATENT SPECIFICATION

(11) **EP 1 615 715 B1**
(45) Date of publication and mention of the grant of the patent: **31.12.2008**
(21) Application number: 04722165.0
(22) Date of filing: 20.03.2004
(51) Int. Cl.: B01D 61/14, A61L 15/60, C08L 39/02, C08F 8/12, C08F 6/12, B01D 63/16

(54) **PROCESS FOR REDUCING THE CONTENT OF WATER-SOLUBLE SALTS OF AQUEOUS SOLUTIONS OF POLYMERS CONTAINING VINYLAMINE GROUPS**
PROZESS ZUR REDUZIERUNG DES GEHALTS WASSERLÖSLICHER SALZE IN WÄSSRIGEN VINYLAMIN-POLYMERLÖSUNGEN
PROCEDE DESTINE A REDUIRE LA TENEUR EN SELS HYDROSOLUBLES DE SOLUTIONS AQUEUSES DE POLYMERES CONTENANT DES GROUPES VINYLAMINE

(30) Priority: 27.03.2003 US 457956 P
(43) Date of publication of application: 18.01.2006
(73) Proprietor: BASF SE, 67056 Ludwigshafen (DE)
(72) Inventor: BENNETT, Andrea, Karen, 56/39 Railway Villa, Chol Buri 20150, Amphur Bang Lamung (TH); MITCHELL, Michael, A., Waxhaw, NC 28173 (US); CARRICO, Peter, W., West Point, MS 39773 (US)
(86) International application number: PCT/EP2004/002928
(87) International publication number: WO 2004/085041

(56) References cited:
- WO-A-00/63295
- US-A- 5 766 478
- US-A- 5 981 689
- US-A- 5 985 160

## Description

The invention relates to a process for reducing the content of water-soluble salts of aqueous solutions of polymers containing vinylamine groups by ultrafiltration and use of the polymers in the manufacture of multicomponent superabsorbent gels.

WO-A-00/67884 discloses a method for fractionation of water-soluble or dispersible synthetic polymers containing amino groups and having a broad molar mass distribution by means of ultrafiltration. The polymer solution or dispersion which is to be fractionated is continuously fed into an ultrafiltration circuit comprising at least one ultrafiltration unit. The retentate with a narrower molar mass distribution and the permeate are continuously discharged in such a way that ultrafiltration circuit is placed in a substantially stationary state. The retentate is preferably used as retention aid, dewatering aid, flocculant and fixing agent in paper production.

Ultrafiltration can also be used to remove salts such as sodium formate or sodium chloride from aqueous polymer solutions, cf. Example 1 of U.S. Patent 5,981,689. Aqueous polyvinylamine solutions having a polymer content of 4% by weight and which are free of sodium formate are obtained. However current ultrafiltration technologies are not particularly successful, because they require the addition of a lot of wash water and do not allow concentration of the polymer solution to a high solids content.

WO-A-02/08302 relates to a method for producing aqueous solutions with a low salt content from polymers containing vinylamine units. The method involves treating aqueous solutions of vinylamine units containing polymers which are obtained by hydrolysis of polymers containing N-vinylformamide units, with a solvent mixture of (a) acetone and (b) an alcohol from the group comprising methanol, ethanol, n-propanol, isopropanol and mixtures thereof, in a weight ratio (a) : (b) from 1:1 to 10 : 1, whereby 0.05 to 0.5 parts by weight of the aqueous polymer solution is used to 1 part by weight of the solvent mixture. The main disadvantage of this process is the high amount of solvent required.

U.S. Patent 6,072,101 relates to multicomponent superabsorbent gel particles which comprise at least one acidic water-absorbing resin and at least one basic water-absorbing resin. Each particle contains microdomains of the acidic resin and/or the basic resin dispersed throughout the particle. Preferred basic resins include lightly crosslinked polyvinylamine and polyethyleneimine, whereas the preferred acidic resin is a lightly crosslinked polyacrylic acid. The absorption capacity of superabsorbent gel particles for electrolyte containing water is dramatically lower than for deionized water. This dramatic decrease in absorption is termed "salt poisoning". It is therefore advantageous for the preparation of multicomponent superabsorbent polymers to combine a salt-free basic superabsorbent polymer or a basic superabsorbent polymer which has only a low electrolyte content with an acid superabsorbent polymer to avoid or minimize the salt poisoning effect.

It is an object of the invention to provide a process for reducing the content of water-soluble salts of aqueous solutions of polymers containing vinylamine groups at a concentration of less Mean 10 % by weight based on total solids in the polymer solution with practically no loss of polyamine.

The object of the invention is achieved by a process for reducing the content of water-soluble salts of aqueous solutions of polymers containing amino groups by ultrafiltration in diafiltration mode to achieve a residual salt content of 1 to 10 % by weight based on total solution solids in the polymer solution comprising feeding an aqueous salt-containing solution of vinylamine group containing polymers at a polymer concentration of at least 7 % by weight to an ultrafiltration unit and removing the water-soluble salts with the permeate from the feed solution by adding thereto less than 4 parts by weight of water per one part by weight of the feed solution, wherein the efficiency of the ultrafiltration unit, which is defined as the ratio of salt in the permeate and in the retentate, is greater than 100 %.

Preferably no more than three parts by weight of water are added to one part by weight of the feed.

The ultrafiltration unit may be composed of spiral wound polymer membranes or vibratory shear membranes. A combination of the said membranes may also be used, for example, a spiral wound polymer membrane together with a vibratory shear membrane. The ultrafiltration unit is preferably composed of vibratory shear membranes.

Such membranes and the other membranes specified above may have a molecular weight cut off of at least 300. It is preferred that the membranes of the ultrafiltration units have a molecular weight cut off of at least 2,000. More the membranes of the ultrafiltration units have a molecular cut off of at least 4,000. For example, the membranes of the ultrafiltration units may have a molecular weight cut off of from 9,000 to 100,000.

Aqueous solutions of polymers containing vinylamine groups are obtained from N-vinylformamide by polymerization alone or together with other monomers and subsequent hydrolysis of the polymerized N-vinylformamide groups of the homo or copolymers, cf. US-A-4,421,602; US-A-5,334,287; EP-B-216,387; US-A-5,981,689, WO-A-00/63295 and US-A-6,121,409. As shown below, all of the polymerized vinylformamide groups in a polymer may be hydrolyzed to vinylamine groups.

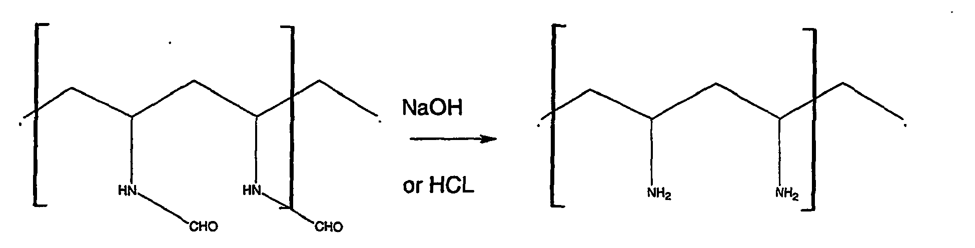

It is also possible that only a certain amount of polymerized N-vinylformamide groups may be hydrolyzed. A degree of hydrolysis of greater than 95 % is, for example, achievable using sodium hydroxide or hydrochloric acid in the hydrolysis step. If sodium hydroxide is used, sodium formate (HCO₂Na) is the by-product of the hydrolysis step. This salt impurity is of minimal concern when the salts do not affect the performance properties of the polymer.

Unfortunately with a superabsorbent polymer (SAP), the presence of sodium formate decreases the adsorption capacity of the SAP dramatically. Therefore a reduction of the content of sodium formate or other water-soluble salts in the aqueous polymer solution to minimal levels is desirable.

Typical aqueous polyvinylamine (PVAm) solutions contain PVAm (obtained from poly N-vinylformamide with a degree of hydrolysis of 95 %) having the following molecular weights or K values according to H. Fikentscher (measured in 5 % strength by weight sodium chloride solution at a temperature of 25°C, a polymer concentration of 0.5 % by weight and pH of 7.0):

| | Mw | K value |
|---|---|---|
| PVAm 1 | 250,000D | 90 |
| PVAm 2 | 120,000D | 70 |
| PVAm 3 | 30,000D | 50 |

The above aqueous PVAm solutions are produced as an aqueous solution of, for example, 8% by weight polyvinylamine solids and 13% by weight sodium formate. However, higher solids can be achieved -12% by weight PVAm and 18% by weight sodium formate. Sodium formate removal is achieved by a washing process - diafiltration. Fresh water is added to the formate containing PVAm solution, which is then pumped across a membrane surface. Pressure forces water and low molecular weight species through the membranes (i.e. the permeate), whilst high molecular weight polymer is retained on the other side of the membranes (i.e. the retentate). The pressure applied to the feed solution is, for example, from 2 to 35 bar, preferably from 15 to 20 bar.

The theory of ultrafiltration states that during diafiltration, micro solutes (ions and low molecular weight polymers) freely permeate through the membranes and maintain the same concentration on both sides of the membrane. The concentration of membrane permeable species (sodium formate) remaining in the feed solution can be calculated by the following:

logₑ(Cₒ/C_{f}) = V_{d}/Vₒ

where: Cₒ is the initial concentration of sodium formate
C_{f} is the final concentration of sodium formate
V_{d} is the volume of fresh water added during diafiltration
Vₒ is the volume of feed in the tank

V_{d}/Vₒ is therefore the number of wash volumes that have been completed. One wash cycle (or wash volume) means the addition of an equivalent amount of fresh water to the starting solution volume and removal of the same amount of permeate solution. According to the equation, 95% of the initial sodium formate is removed after 3 wash volumes and 98% of the initial formate is removed after 4 wash volumes have been completed. Therefore to achieve the desired degree of purity for this process 4 - 5 wash cycles are normally required. It is desirable to minimize the wash volumes required to reach purity, as this significantly reduces the wastewater costs. The salt content of the aqueous solution is reduced, for example, to 1 to 10, preferably 2 to 4 % by weight of residual sodium formate, based on total solution solids in the polymer solution.

Once the correct level of purity is achieved, the PVAm solution is then concentrated to the maximum amine solids achievable with each technology (before the viscosity of the solution prohibits further concentration). Diafiltration and concentration together constitute ultrafiltration.

Other important operating parameters include permeate flux rate (rate of fluid transport) across the membranes. The greater the flux rate, the smaller the membrane area required to purify the solution. Amine loss across the membranes should also be minimized by careful choice of membrane.

There are different ultrafiltration technologies for desalting PVAm solutions, namely

**Standard Spiral Wound Polymer Membranes -** which resemble a rolled-up piece of paper, with two flat rectangular pieces of membrane held apart by a web spacer and sealed along three sides. The unsealed side of the rectangle is attached to a central tube and then rolled up. The membrane is then placed inside a tube so that the feed solution can enter one end under pressure and exit the opposite end after flowing past the membrane surfaces. The main advantage of spiral wound membranes is the large surface area achieved in one module, however they are limited by the fact they cannot handle viscous solutions.

**Tubular Membranes** (not in accordance with the invention) - are generally composed of small polymeric tubes, which vary in diameter from 0.5 to 2.5 inches. They are placed within a rigid supporting tube that is usually metallic. The advantage of these systems is their tolerance to suspended solids in the feed stream - provided the feed is pumped rapidly along the membrane pathway. Therefore large pumps are required which can be expensive. However tubular membranes can handle very thick solutions and also have long life times.

**Vibratory Shear Membranes -** The company New Logic in the USA offers a new technology that utilizes Vibratory Shear Enhanced Processing (VSEP) or vibrating disc technology to allow processing at very high solids and flux rates. In an industrial VSEP machine, the membrane elements are arranged as parallel disks. The disk stack is vibrated in a torsional oscillation which focuses shear waves at the membrane surface, thus repelling solids and foulants within the gel boundary layer.

Ultrafiltration can be carried out in batch and in continuous mode. It was found that batch mode gave the same flux readings as continuous mode but formate removal was more efficient in batch mode. The temperature of the aqueous polymer solutions to be purified may be in the range of from 20 to 95°C, preferably from 50 to 70°C. The water-soluble salts to be removed according to the invention from the polymer solution are selected from the group consisting of alkali metal salts, ammonium salts such as ammonium chloride and alkali earth metal salts. Since the vinylamine groups containing polymers are in most cases obtained by hydrolysis of homo and/or copolymers of N-vinylformamide in the presence of sodium hydroxide or hydrogen chloride, the reduction of the content of sodium formate or sodium chloride from aqueous solutions of vinylamine groups containing polymers is a preferred embodiment of the invention.

The aqueous polymer solutions obtained by ultrafiltration have, for example, a polymer concentration of from 8 to 35, preferably of from 25 to 30 % by weight. It is preferred to use the solution obtained according to the invention for the manufacture of polyvinylamine based superabsorbent gets or multi-component polymers.

Polyvinylamine-based superabsorbent gels are disclosed in U.S. Patent 5,981,689, column 2 line 65 to column 15, line 44 and in the claims as well as in U.S. Patent 6,121,409, column 3, line 9 to column 18, line 6.

Multicomponent superabsorbent gels are disclosed in U.S. Patent 6,222,091, column 4, line 49 to column 46, line 43. Particles of multicomponent SAP comprise at least one basic water-absorbing resin and at least one acidic water-absorbing resin. Each particle contains at least one microdomain of the acidic resin in contact with, or in close proximity to, at least one microdomain of the basic resin. The multicomponent SAP can for example have the form of granules, fibers, powder, flakes, films, or foams. The weight ratio of the acidic to the basic resin in the multicomponent SAP may from about 90 : 10 to about 10 : 90. preferably from 30 : 70 to 70 to 30. The acid and the basic SAP may be partially neutralized, e.g. up to 25 % by mole but are preferably not neutralized.

The polyvinylamine solution obtained by ultrafiltration may be crosslinked to form a gel which can be mixed with a polyacrylic acid gel to form a multicomponent superabsorbent gel (MDC gel). Crosslinking of the polyvinylamine solution may, for instance, be carried out on a continuous belt with the application of thermal energy or microwave energy or may also be processed in a Buss Reactotherm, i.e. a single shaft continuous kneder. The crosslinking step of the polyvinylamine may also be carried out batchwise. The crosslinked PVAm is water-insoluble and is water-swellable.

In order to prepare a MDC gel the crosslinked PVAm is mixed with a SAP having preferably a degree of neutralization of 0 %. The production of unneutralized SAP gel may be carried out according to prior art methods, for example, in a List ORP Reactor using redox initiation of the monomers, or an a continuous belt whit photoinitiation or with redox initiation.

The mixing of the two different polymer gels may be carried out in usual apparatuses such as Buss Reactotherm, Readco Extruder (a twin shaft high shear continuous extruder), Brabender Extruder (a twin screw unit, counter rotating), a batch kneader or a List ORP Reactor. The mixed gel, i.e. the MDC gel, is discharged from the mixing devices in a granulated form. The granules have, for example, a solids content of 20 to 40, preferably 25 to 35 % by weight.

The MDC granules can be dried on conventional driers, for example, band drier, high air flow flash driers such as ring drier, fluid bed drier, Bepex Soldaire Dryer ring drier and fluid bed drier. The dried MDC granules may be milled on a roller mill and sieved by standard technique.

The K value of the polymers was determined according to H. Fikentscher, Cellulose-Chemie, Vol. 13, 58 -64 and 71 - 74 (1932) in 5 % strength by weight sodium chloride solution at a temperature of 25°C, a polymer concentration of 0.5 % by weight and pH of 7.0

### Examples

All trials were performed on ultrafiltration (UF) pilot units in batch mode at a temperature of 60°C. A pressure of 4 bar was exerted on the feed solution in Unit 1 and 8 bar in Units 2 and 3. The following units were used:
Unit (1) was fitted with a spiral membrane having a membrane area of 33 m² from PCI. Unit (2) was fitted with a tubular membrane having a membrane area of 11.6 m² from PCl. The molecular weight cut off (MWCO) of the membranes used in Units (1) and (2) was 9,000 Dalton.
   Unit (3) was fitted with vibratory shear membranes from New Logic Corporation having a membrane area of 1.4 m² and a MWCO of either 10,000 Dalton, 400 Dalton or nanofiltration membrane with a 10 % salt reject rating depending on the molecular weight of the polymers. Polymers having K values of 70 and higher were diafiltrated with membranes of 9,000 or 10,000 Dalton MWCO and polymers of K value of 50 were diafiltrated with membranes of 400 Dalton MWCO or nanofiltration membrane with 10 % by weight salt reject rating.

### Example 1

### Flux Rates

The following table details the various flux rates achieved with the different solutions at different PVAm solids levels. Flux rates are measured in I/m²h - litres (of permeate) per square metre (of membrane area) per hour.

**Table 1**

| **Feed Solution K value** | **Vibratory - Unit (3)** | **Tubular-Unit** (2) | **Spiral - Unit (1)** |
|---|---|---|---|
| 8% PVAm solids 50 | 34 - 38 I/m²h | 14 - 16 I/m²h | 2.7 - 3.4 I/m²h |
| 8% PVAm solids 70 | 14 -17 I/m²h | 4 I/m²h | - |
| 4% PVAm solids 70 | - | - | 10 I/m²h |
| 8% PVAm solids 90 | 12,4 I/m²h | 4 l/m²h | - |
| 4% PVAm solids 90 | - | - | 10 l/m²h |

### Solids Achievable

Table 2 shows the PVAm solids achievable following the diafiltration (washing) and concentration steps.

**Table 2**

| **Feed Solution K value** | **Vibratory - Unit (3)** | **Tubular-Unit (2)** | **Spiral - Unit (1)** |
|---|---|---|---|
| PVAm 50 | 25% | 21 % | 16 -17% |
| PVAm 70 | 12.7% | 10% | - |
| PVAm 90 | 12% | 10% | - |

It can be seen that the UF Unit (3) offers benefit in the flux rate achieved (meaning less membrane area is required in a full size plant to purify the same volume of solution) and the solids concentration achieved.

### Example 2

### Amine Loss Through the Membranes

Several different membranes have been trialed with the three types of Ultrafiltration systems. Amine loss was found to be negligible, even when using the solution of PVAm having a K value of 50. Amine loss when using a PVAm solution of this molecular weight (30.000D) was 0,002% for UF Unit (3) and 0,009% for UF Units (2) and (1).

### Example 3

### Efficiency of Formate Removal

The standard theory of diafiltration says that equilibrium is achieved between the permeate and feed solutions across the membrane. 100% passage of formate across the membrane means no sodium formate is retained by the membrane or the gel layer that builds up on the membrane surface. This is equivalent to 100% efficiency of formate removal (based on the theory of diafiltration) and is the target.

According to the present invention it is necessary to achieve greater than 100% efficiency of sodium formate removal i.e. the situation where formate passage is greater 100% and the formate concentration is greater in the permeate than in the retentate. This is equivalent to a reverse osmotic effect and is usually observed at high sodium formate concentrations.

Greater than 100% efficiency means less wash volumes will be required to achieve the same level of impurity - thus speeding up production rate and producing less waste water. Table 3 details the efficiency of formate removal for the theoretical situation of 100% efficiency and for the observed efficiencies for the various Ultrafiltration systems that were tested. Tubular Membranes - Unit (2) were used as Comperative Examples, showing an efficiency lower than 100 %. The results are all based on a starting solution of 13% sodium formate and 8% PVAm.

**Table 3**

| No. Of Wash Vol. Completed | Theoretical Efficiency | Spiral Membranes - Unit (1) | Tubular Membranes - Unit (2) | Vibratory Membranes - Unit (3) |
|---|---|---|---|---|
| 1 | 100% | 115% | 95% | 128% |
| 2 | 100% | 114% | 90% | 127% |
| 3 | 100% | 100% | 70% | 115% |
| 4 | 100% | 100% | 70% | - |

Therefore it can be seen that the efficiency of formate removal decreases with decreasing sodium formate concentration for each Ultrafiltration system. However Unit (3) achieves surprisingly better than 100% formate passage, which means that it is extremely effective in removing sodium formate.

**Table 4**

| No. of Wash Vol. Completed | % Formate Remaining in Solution (based on total solids) | | | |
|---|---|---|---|---|
| | Theoretical Passage of 100% | Spirals - Unit (1) | Tubular - Unit (2) | Vibratory - Unit (3) |
| 0 | 61,9% | 61,9% | 61,9% | 61,9% |
| 1 | 37,4% | 34% | 38,6% | 31,1% |
| 2 | 18% | 14,2% | 20,3% | 11,3% |
| 3 | 7,5% | 5,8% | 11,7% | 3,8% |
| 4 | 2,9% | 2,2% | 5,9% | - |
| 5 | 1,1% | - | 3% | |

It can be seen from the results in Table 4 that 4 wash volumes would normally be required to achieve the desired level of purity and this is the case when using the spiral membranes of UF Unit (1). However the tubular membranes i.e. UF Unit (2) achieved worse efficiency and 5 wash volumes had to be completed.
The trials with the New Logic system, i.e. UF Unit (3) were extremely successful and only 3 wash volumes were required to remove the desired amount of sodium formate (less than 4 % by weight with reference to the total solids).

## Claims

1. A process for reducing the content of water-soluble salts of aqueous solutions of polymers containing vinylamine groups by ultrafiltration in diafiltration mode to achieve a residual salt content of 1 to 10 % by weight based on total solution solids in the polymer solution, **characterized in that** the process comprises feeding an aqueous salt-containing solution of vinylamine group containing polymers at a polymer concentration of at least 7 % by weight to an ultrafiltration unit and removing the water-soluble salts with the permeate from the feed solution by adding thereto less than 4 parts by weight of water per one part by weight of the feed solution, wherein the efficiency of the ultrafiltration unit, which is defined as theratio of salt in the permeate and in the retentate, is greater than 100%.

2. The process of claim 1 wherein the ultrafiltration unit is composed of spiral wound polymer membranes or vibratory shear membranes.

3. The process of claim 1 or 2 wherein no more than three parts by weight of water are added to one part by weight of the feed.

4. The process of claims 1 to 3 wherein the ultrafiltration unit is composed of vibratory shear membranes.

5. The process of claims 1 to 4 wherein the ultrafiltration unit is composed of vibratory shear membranes having a molecular weight cut off of at least 300.

6. The process of claims 1 to 5 wherein the membranes of the ultrafiltration units have a molecular weight cut off of at least 2,000.

7. The process of claim 1 to 6 wherein the membranes of the ultrafiltration units have a molecular cut off of at least 4,000.

8. The process as claimed in claim 1 to 7 wherein the membranes of the ultrafiltration units have a molecular weight cut off of from 9,000 to 100,000.

9. The process of claims 1 to 8 wherein the water-soluble salts are selected from the group consisting of alkali metal salts, ammonium salts and alkali earth metal salts.

10. The process of claim 9 wherein the water-soluble salts are selected from the group consisting of sodium formate and sodium chloride.

11. The process of claims 1 to 10 wherein the polymers containing vinylamine groups are obtained by hydrolysis of homo and/or copolymers of N-vinylformamide.

## Patentansprüche

1. Verfahren zur Verringerung des Gehalts wäßriger Lösungen von Vinylamingruppen enthaltenden Polsmerisaten an wasserlöslichen Salzen durch Ultrafiltration im Diafiltrationsmodus zur Erzielung eines Salzrestgehalts von 1 bis 10 Gew.-%, bezogen auf die gesamten Lösungsfeststoffe in der Polymerisatlösung, **dadurch gekennzeichnet, daß** man eine wäßriges Salz enthaltende Lösung von Vinylamingruppen enthaltenden Polymerisaten bei einer Polymerisatkonzentration von mindestens 7 Gew.-% einer Ultrafiltrationseinheit zuführt und die wasserlöslichen Salze mit dem Permeat aus der Einsatzlösung entfernt, indem man diese mit weniger als 4 Gewichtsteilen Wasser pro Gewichtsteil Einsatzlösung versetzt, wobei die Effizienz der Ultrafiltrationseinheit, die als das Verhältnis von Salz im Permeat und im Retentat definiert ist, größer als 100% ist.

2. Verfahren nach Anspruch 1, bei dem die Ultrafiltrationseinheit aus Spiralwickelpolymermembranen oder Vibrationsschermembranen besteht.

3. Verfahren nach Anspruch 1 oder 2, bei dem man höchstens drei Gewichtsteile Wasser pro Gewichtsteil des Einsatzes zugibt.

4. Verfahren nach den Ansprüchen 1 bis 3, bei dem die Ultrafiltrationseinheit aus Vibrationsschermembranen besteht.

5. Verfahren nach den Ansprüchen 1 bis 4, bei dem die Ultrafiltrationseinheit aus Vibrationsschermembranen mit einem Molekulargewichts-Cutoff von mindestens 300 besteht.

6. Verfahren nach den Ansprüchen 1 bis 5, blei dem die Membranen der Ultrafiltrationseinheiten einen Molekulargewichts-Cutoff von mindestens 2.000 aufweisen.

7. Verfahren nach den Ansprüchen 1 bis 6, bei dem die Membranen der Ultrafiltrationseinheiten einen Molekulargewichts-Cutoff von mindestens 4.000 aufweisen.

8. Verfahren nach den Ansprüchen 1 bis 7, bei dem die Membranen der Ultrafiltrationseinheiten einen Molekulargewichts-Cutoff von 9.000 bis 100.000 aufweisen.

9. Verfahren nach den Ansprüchen 1 bis 8, bei dem man die wasserlöslichen Salze aus der Gruppe bestehend aus Alkalimetallsalzen, Ammoniumsalzen und Erdalkalimetallsalzen auswählt.

10. Verfahren nach Anspruch 9, bei dem man die wasserlöslichen Salze aus der Gruppe bestehend aus Natriumformiat und Natriumchlorid auswählt.

11. Verfahren nach den Ansprüchen 1 bis 10, bei dem die Vinylamingruppen enthaltenden Polymerisate durch Hydrolyse von Homo- und/oder Copolymerisaten von N-Vinylformamid erhalten werden.

## Revendications

1. Procédé servant à réduire la teneur en sels solubles dans l'eau de solutions aqueuses de polymères contenant des groupes vinylamine par ultrafiltration en mode diafiltratlon pour atteindre une teneur en sel résiduelle de 1 à 10 % en poids sur la base des matières solides en solution totales dans la solution de polymères, **caractérisé en ce que** le procédé comprend les étapes consistant à introduire dans une unité d'ultrafiltration une solution aqueuse de polymères contenant des groupes vinylamine contenant des sels à une concentration en polymères d'au moins 7 % en poids et à enlever avec le perméat les sels solubles dans l'eau de la solution introduite en y ajoutant moins de 4 parties en poids d'eau pour une partie en poids de la solution introduite, dans lequel le taux d'efficacité de l'unité d'ultrafiltration, lequel est défini comme étant le rapport du sel dans le perméat sur celui dans le rétentat, est supérieur à 100 %.

2. Procédé de la revendication 1 dans lequel l'unité d'ultrafiltration est composée de membranes polymériques en spirale ou de membranes vibrantes VSEP.

3. Procédé de la revendication 1 ou 2 dans lequel pas plus de trois parties en poids d'eau sont ajoutées pour une partie en poids de la charge introduite.

4. Procédé des revendications 1 à 3 dans lequel l'unité d'ultrafiltration est composée de membranes vibrantes VSEP.

5. Procédé des revendications 1 à 4 dans lequel l'unité d'ultrafiltration est composée de membranes vibrantes VSEP ayant un seuil de coupure d'au moins 300.

6. Procédé des revendications 1 à 5 dans lequel les membranes des unités d'ultrafiltration ont un seuil de coupure d'au moins 2 000.

7. Procédé des revendications 1 à 6 dans lequel les membranes des unités d'ultrafiltration ont un seuil de coupure d'au moins 4 000.

8. Procédé selon les revendications 1 à 7 dans lequel les membranes des unités d'ultrafiltration ont un seuil de coupure allant de 9 000 à 100 000.

9. Procédé des revendications 1 à 8 dans lequel les sels solubles dans l'eau sont sélectionnés dans le groupe constitué de sels de métaux alcalins, de sels d'ammonium et de sels de métaux alcalinoterreux.

10. Procédé de la revendication 9 dans lequel les sels solubles dans l'eau sont sélectionnés dans le groupe constitué du formiate de sodium et du chlorure de sodium.

11. Procédé des revendications 1 à 10 dans lequel les polymères contenant des groupes vinylamine sont obtenus par hydrolyse d'homopolymères et/ou de copolymères du *N*-vinylformamide.
